# FASCICULE DE BREVET EUROPEEN

(11) **EP 2 119 223 B1**
(45) Date de publication et mention de la délivrance du brevet: **09.11.2011**
(21) Numéro de dépôt: 08761814.6
(22) Date de dépôt: 30.01.2008
(51) Int. Cl.: H04N 5/232, A61B 3/113

(54) **PROCEDE DE COMMANDE A PARTIR D'UN SIGNAL OCULAIRE VOLONTAIRE, NOTAMMENT POUR UNE PRISE DE VUES**
STEUERVERFAHREN AUF DER BASIS FREIWILLIGER AUGENSIGNALE, IM BESONDEREN FÜR FILMAUFNAHMEN
CONTROL METHOD BASED ON A VOLUNTARY OCULAR SIGNAL, PARTICULARLY FOR FILMING

(30) Priorité: 02.02.2007 FR 0700754
(43) Date de publication de la demande: 18.11.2009
(73) Titulaire: Binocle, 94360 Bry-sur-Marne (FR); UNIVERSITE PIERRE ET MARIE CURIE (PARIS 6), 75252 Paris Cedex 05 (FR); Universite Paris VIII, 93526 Saint Denis Cedex 02 (FR); Centre National de la Recherche Scientifique, 75016 Paris (FR)
(72) Inventeur: BOUCHON-MEUNIER, Bernadette, F-75015 Paris (FR); PUPULIN, Yves, F-75011 Paris (FR); BACCINO, Thierry, F-06000 Nice (FR); TIJUS, Charles, F-75003 Paris (FR)
(74) Mandataire: Priori, Enrico
(86) Numéro de dépôt international: PCT/FR2008/000106
(87) Numéro de publication internationale: WO 2008/107564

(56) Documents cités:
- EP-A- 1 168 830
- US-A- 4 109 145
- US-A- 5 583 795
- US-A- 5 689 619
- US-A- 5 717 413
- US-A- 5 751 260
- US-A- 5 790 099
- US-A1- 2002 180 799

## Description

La présente invention concerne un procédé de commande à partir d'un signal oculaire, notamment utilisable pour une commande reliée à un dispositif de prise de vues à au moins une caméra réelle ou virtuelle.

Les mouvements de l'oeil sont des mouvements de faible amplitude (1 à 25 minutes d'arc), de très courte durée (0,01 à 0,05 seconde) et qui ont une vitesse angulaire qui peut atteindre 500°/s. Depuis plus de dix ans, on utilise les caméras vidéo pour recueillir le parcours oculaire et l'analyser. L'analyse du parcours oculaire par le traitement des données oculaires permet de repérer les déplacements oculaires de nature involontaire que sont les micro-tremblements de l'oeil (nystagmus), les dérives lentes du regard, les clignements, les déplacements oculaires de nature volontaire (changement des coordonnées XY de la position de l'oeil sur l'écran), la vitesse des mouvements, la taille des saccades, les fixations oculaires et leur durée et les mouvements de vergence.

Le recueil et l'analyse du mouvement oculaire à l'aide d'un oculomètre, précédés d'une calibration nécessaire pour déterminer les coordonnées XY de la position du regard sur l'écran, sont très fiables, rapides et de grande précision. Ils permettent d'avoir en temps réel le parcours oculaire associé à l'inspection du contenu d'un écran, notamment pour étudier l'intérêt porté aux composantes d'une scène visuelle.

L'intérêt de la commande oculaire de dispositifs a été relevé très tôt (Ware & Mikaelian, An évaluation of an eye tracker as a device for computer input CHI +GI, 1987 - Conférence Proceedings - SIGHI Bulletin ACM, p. 183-188), pour des applications militaires (par example Smyth, Bates, Lopez & Ware, "A comparison of eye-gaze to touch panel and head fixed reticle for helicopter display control" - Proceeding of the 2^{nd} Mid-Atlantic Human Factors Conférence 1994, p. 49 et suivantes"), les interactions humain-machine (par exemple RJK Jacob "The use of eye movements in human-computer interaction techniques - what you look is what you get" ACM Transactions on Information Systems 1991, 9(3), p. 152-169), avec la commande à distance (par exemple : projet du "IBM Almaden Research Center"), ou la commande robotique (par exemple : Atienza & Zelinsky, 2003 "Interactive skills using active gaze tracking" - Proceedings of the 5th International Conference on multimodal interfaces, Vancouver- Canada, p. 188-195).

Le mouvement oculaire, lorsqu'il est opératif, est plus rapide parce que plus direct que la commande motrice, ceci pour les commandes de pointage et de direction (travaux du "Gaze-Based Interaction Group").

Ainsi, une caméra à haute résolution placée à deux mètres d'une personne peut suivre les mouvements de sa rétine et les traduire en commandes oculaires. Et il existe :
- des dispositifs pour saisir des caractères alphabétiques à partir de l'analyse des fixations oculaires sur des claviers numériques,
- des dispositifs de commande par le regard plus intégrés (gestion des courriels électroniques, écriture de textes, comme le projet "Eye Gaze Interaction" et le système "Visuoboard")
- et des dispositifs d'analyse du comportement sur des objets (filmés par une caméra) couplée à l'analyse du suivi du regard (Atienza & Zelinsky, 2003).

Le principe de tous ces dispositifs repose sur l'analyse d'une fixation oculaire d'une zone de l'écran (« location-based interaction ») en positionnant le curseur lorsqu'une fixation oculaire est détectée. La commande s'effectue alors par un clignement de l'oeil, par une commande manuelle (clic de la souris), ou encore par une durée longue de la fixation lorsqu'il s'agit de zoomer par exemple, ou d'ouvrir un menu, associée à une durée de la fixation oculaire (« time-based interaction »).

Ce principe ne permet pas la commande continue nécessaire pour mettre en ouvre les fonctions de pilotage d'une caméra réelle ou virtuelle (zoom, mouvements de la caméra, ...).

Les mouvements des yeux et les logiciels d'analyse du parcours du regard présentent trois caractéristiques qui expliquent pourquoi la commande oculaire s'est jusqu'ici limitée aux commandes oculaires qui ne considéraient pas le mouvement continu de l'oeil comme un effecteur de commandes. Cette commande oculaire était alors réalisée à partir d'icônes (« location-based interaction »), de boutons ou d'emplacements bien définis.

La première caractéristique est que l'oeil capture de l'information et qu'il n'en produit pas. Lorsque les yeux font partie d'un système de commande, il faut distinguer ce qui relève de la prise d'information de ce qui relève de la commande. Toute méprise (produire une commande lorsque l'utilisateur regarde ou considérer qu'il regarde alors qu'il veut produire une commande) rend le système de commande peu fiable et difficile à utiliser. Une commande peut être, par exemple, une fixation suffisamment longue suivie d'un clignement des paupières.

La seconde caractéristique est que les yeux sont toujours en mouvement (des micros mouvements que sont les nystagmus jusqu'aux saccades oculaires d'amplitude variée). Il faut donc pouvoir bien distinguer ce qui relève des fixations de ce qui relève des déplacements oculaires. C'est ce que font tous les logiciels d'analyse de données oculaires qui agrègent les positions du regard proches spatialement et temporellement. Ils définissent ainsi des zones qui correspondent aux fixations oculaires.

La troisième caractéristique est que les logiciels d'analyse ne fournissent, en sortie, que les fixations oculaires (localisation et durée) et les saccades (localisation de départ, localisation d'arrivée, amplitude et durée). Les déplacements lents et orientés du regard dans une direction ne sont pas pris en compte par les logiciels d'analyse.

Mécanique ou logicielle, la commande d'une caméra est toujours motrice, si bien qu'il faut gérer manuellement la caméra et par le regard ce qui est filmé.

II n'existe pas de système de commande oculomotrice de prise de vues 2D, XY, alors même qu'un tel système présenterait d'indéniables avantages. Parmi les avantages, on citera la possibilité d'une commande mains libres, mais surtout la commande de la prise de vues d'une image à partir de l'image elle-même dans laquelle la manière naturelle d'inspecter une scène visuelle est appliquée à la prise de vues. Un tel dispositif permettrait par exemple à un réalisateur disposant d'un viseur et d'un écran de contrôle de travailler directement la prise de vues à partir d'un écran de contrôle de grande taille. Cet écran de grande taille présenterait trois avantages:
- une plus grande liberté de mouvement du regard de l'opérateur,
- une plus grande précision dans l'analyse de la détection du mouvement de l'oeil,
- une observation de l'image, par l'opérateur, dans une taille plus proche de sa diffusion finale.

Le Brevet US 5 717 413 concerne la mise en oeuvre d'une commande à partir de la détection d'un mouvement lent produit naturellement lors de l'inspection de scènes usuelles.

Le Brevet US 4 109 145 concerne une technique de suivi selon laquelle un opérateur fixe son regard sur un symbole ou une icône, et la commande est effectuée par une fixation et non par un mouvement lent.

La technique décrite dans la Demande US 2002/180 799 prévoit de détecter seulement la position du regard et non une information de vitesse.

L'idée de base de la présente invention est d'utiliser le regard comme système de commande, notamment pour le pilotage d'une caméra réelle ou virtuelle 2D et/ou 3D, à partir de l'analyse des mouvements lents et orientés du regard.

L'invention concerne ainsi un procédé de commande à partir d'un signal oculaire de commande tel que défini dans la revendication 1.

Avantageusement, le dit mouvement lent correspond à un déplacement angulaire régulier de l'oeil dont la vitesse angulaire est comprise entre 1 degré par seconde et 40 degrés par seconde, notamment entre 1°/s et 30°/s, et plus particulièrement entre 5 et 20 °/s.

La détection du mouvement lent peut comprendre la détermination de son trajet, notamment de sa direction, par un procédé de calcul, notamment par régression linéaire.

La détection d'une saccade (ou mouvement rapide) et/ou d'une fixation peut produire une interruption de la génération du signal de commande.

Le signal oculaire de commande peut être une fonction (croissante ou décroissante) du déplacement angulaire. Le signal oculaire de commande peut représenter la position de l'oeil et/ou la vitesse du mouvement lent de l'oeil et/ou sa direction. De cette façon, dès qu'un mouvement lent est détecté, tous les paramètres de celui-ci peuvent être utilisés pour gérer une commande continue, son début et/ou sa fin.

Au moins un paramètre de la commande, par exemple une vitesse, peut être fonction d'au moins un paramètre du déplacement oculaire, par exemple sa vitesse et/ou sa direction.

Une commande peut, par exemple, consister en un choix entre des options. Ces options peuvent être réparties selon un continuum ou des valeurs discrètes en fonction d'au moins un paramètre du déplacement oculaire. Par exemple, tant que le mouvement lent se poursuit, le choix reste ouvert, et l'arrêt du mouvement lent ou son interruption pendant une durée supérieure à un seuil choisi fixe le choix.

Le début et/ou la fin de la commande peut être généré par le début et/ou la fin du signal oculaire de commande, ou bien encore par un ordre vocal, visuel et/ou manuel.

Selon une première variante, le type de commande auquel le signal oculaire de commande est affecté est déterminé par au moins un paramètre du déplacement oculaire, par exemple sa direction.

Selon une deuxième variante, le type de commande auquel le signal oculaire de commande est affecté, est déterminé par un ordre vocal, visuel ou manuel.

Le signal oculaire de commande peut être également un signal par tout ou rien.

L'invention est particulièrement adaptée à la commande à distance de la prise de vues de caméras embarquées ou sur des bras articulés qui est très développée à des fins esthétiques, de télésurveillance, mais aussi d'intervention à distance lorsqu'il s'agit de manipuler des outils à distance. Lorsqu'il s'agit de manipulation à faire à distance, il y a deux systèmes à gérer manuellement: la caméra (si on veut agir sur la prise de vues) et l'instrument que l'on commande. L'invention permet de commander la caméra par le regard et de commander l'instrument manuellement.

L'invention s'applique en particulier à la commande oculaire d'au moins une caméra réelle ou virtuelle.

Le type de commande auquel le signal de commande est affecté peut être, par exemple le démarrage d'une prise de vues et/ou un zoom et/ou un réglage de diaphragme et/ou un réglage de mise au point et/ou une rotation de caméra autour de son axe et/ou un déplacement (« travelling ») et/ou (dans le cas d'un système stéréoscopique) un entraxe et/ou une vergence. II peut être déterminé par un ordre vocal (commande vocale et/ou visuel (fixation sur une icône, clignements, ...) et/ou manuel (clavier, souris, manette de jeu etc.) ou bien par un paramètre du mouvement angulaire, par exemple sa direction.

La classe des mouvements non lents comportera, selon le mode choisi, deux sous-classes:
- une sous-classe de fixations oculaires, et une sous classe de saccades oculaires, définies notamment par un pic d'accélération suivi par une forte décélération, sinon.

D'autres caractéristiques et avantages de l'invention apparaîtront mieux à la lecture de la description ci-après en liaison avec les dessins dans lesquels :
- la figure 1 illustre le fonctionnement de la commande oculaire appliquée à une caméra vidéo,
- la figure 2 illustre des repères de prise de vues,
- la figure 3 représente les données oculaires de base pour deux mouvements lents précédés et séparés par des mouvements non lents. En abscisse, sont portées 4000 positions successives des yeux. Ces données sont échantillonnées toutes les 20 ms, soit une durée d'enregistrement de 80 secondes,
- la figure 4a illustre la corrélation et la pente de régression pour un petit nombre de coordonnées successives d'un mouvement lent (4a). La figure 4b illustre la fonction bivariante relative aux données qui sont associées au mouvement lent. Les figures 5a et 5b illustrent une commande de caméra respectivement à l'aide d'un écran ou d'un verre à matrice active (5a), ou par vision directe d'une scène (5b).

L'invention met en oeuvre la production, le recueil et le traitement de mouvements volontaires, lents et orientés des yeux, qui sont produits avec une lenteur et une orientation que le regard n'a pas dans ses conditions naturelles d'inspection des scènes visuelles. On trouve toutefois cette lenteur et cette orientation (que l'on peut facilement déceler) dans des cas de dérive involontaire de l'oeil vers le bas, hors inspection. Il s'agit des mouvements dits lents, de l'ordre de 1°/s à 40°/s, qui ne sont pas considérés par les logiciels de suivi du regard parce qu'ils ne correspondent ni à des fixations, ni à des saccades. La réalisation de tels mouvements lents, en l'absence de suivi oculaire d'une cible, nécessite un entraînement : les personnes non entraînées n'y parviennent pas : elles font des saccades (cf. Richard J. Krauzlis 'Recasting the Smooth Pursuit Eye Mouvement System'- Journal of Neurophysiology 91 : pages 591-603, 2004, notamment page 599).

L'extraction des mouvements lents et orientés (en fait des micro-saccades qui fournissent un déplacement oculaire dans une même direction) est réalisée selon l'invention par une méthode d'analyse de données. Cette extraction permet de distinguer entre des mouvements intentionnels actifs destinés à agir sur la caméra et des mouvements naturels passifs de suivi. L'analyse, elle, est associée à une adaptation à l'utilisateur pour intégrer automatiquement ses particularités physiologiques et comportementales.

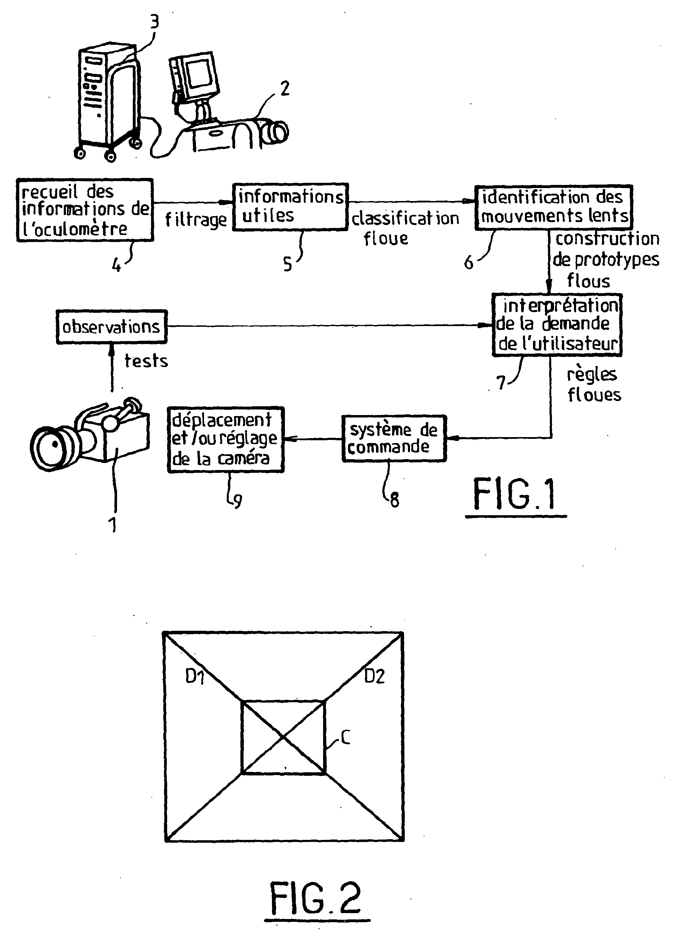

Le système représenté à la figure 1 comporte une caméra 1, un oculomètre 2 et une installation 3 pour recueillir les informations de l'oculomètre. Ces informations sont recueillies en 4. Après filtrage, les informations utiles sont stockées en 5, puis classifiées pour produire une identification des mouvements lents en 6. Une table de correspondance permet en 7 d'interpréter les mouvements lents identifiés pour générer une commande en 8 qui produit en 9 des signaux de déplacement et/ou de réglage de la caméra.

Le procédé est le suivant:
1. un matériel de base qui comprend un oculomètre connu en soi. Cet oculomètre fournit les coordonnées XY de la position du regard sur l'écran où s'affichent la prise de vues caméra et la valeur de vergence des yeux,
2. une phase de calibrage qui a pour buts :
   a. d'ajuster l'enregistrement de la position de chacun des yeux par rapport à des positions XY prédéfinies de l'écran,
   b. de mettre en correspondance la vergence des yeux à des positions en profondeur lorsqu'il s'agit d'oculométrie 3D, de commande de caméras 3D.

   Il s'agit de fixer successivement des points (croix) apparaissant à égale distance sur l'écran. Ces points dessinent la *grille* de calibrage 2D, XY ou 3D, XYZ. Les valeurs enregistrées au moment du calibrage (coordonnées de l'oeil sur des positions prédéfinies) sont utilisées lors de l'analyse des données pour projeter géométriquement la position de l'oeil à chaque instant dans le plan XY de l'écran ou, pour la commande de caméras 3D, dans l'espace XYZ.
3. un affichage à l'écran qui comprend l'image de la prise de vues, deux diagonales D1 et D2 et un cadre centré C (figure 2).
4. des commandes de la caméra 2D ou 3D qui sont affichées à l'écran sous forme d'icônes :
   i. les commandes liées aux optiques de prises de vues (zoom, point, diaphragme),
   ii. les commandes liées aux mouvements de rotation des caméras (panoramique horizontal ("pan") ou vertical ("tilt"), rotation autour de l'axe de la caméra ("roll") et à leurs combinaisons, les mouvements de travelling latéraux, verticaux, dans l'axe et leurs combinaisons,
   iii. et, dans le cas d'une caméra 3D, les commandes liées aux réglages des paramètres du relief pour la profondeur (entraxe et vergence).
5. une fixation oculaire dans la zone correspondant à une commande, ou à une combinaison de commandes qui active cette commande ou cette combinaison de commandes. L'icône correspondant à la commande activée, ou aux commandes activées, est mise en inverse vidéo pour indiquer que les mouvements lents du regard qui seront enregistrés correspondent à cette commande,
6. des coordonnées successives en XY ou en XYZ qui sont analysées en termes de proximité et de direction. On utilise des techniques d'analyse des données basées sur apprentissage pour déterminer en temps réel si les coordonnées successives relèvent d'une saccade (coordonnées distantes), d'une fixation oculaire (coordonnées proches sans direction) ou d'un mouvement lent du regard (coordonnées proches dans une direction approximative),
7. une table de correspondance pour chaque commande de la caméra qui permet de déterminer la valeur de la commande (avant ou arrière, par exemple, pour la commande zoom) à partir de la catégorisation de la direction repérée (valeur x croissant : zoom avant, x décroissant : zoom arrière), dans le cadre de « limites des variations de valeurs », paramétrables, associées à chaque commande,
8. une valeur de la commande (par exemple : « zoom avant ») est transmise au dispositif mécanique de la caméra qui est associée à une valeur de vitesse proportionnelle au déplacement lent du regard,
9. un repérage d'une large saccade ou d'une fixation qui met fin à la commande, sauf en commande « prise de vues ». Une commande permettra de revenir à l'état initial qui précédait l'exécution de la commande.
10. Les conditions de réalisation du procédé sont les suivantes :
   a. chaque commande est paramétrable. L'utilisateur détermine la relation entre la vitesse d'exécution de la commande et le déplacement lent de son regard,
   b. chaque commande est adaptable. Le système tient compte des réussites et échecs pour l'exécution des commandes,
11. une phase d'apprentissage permet de paramétrer le dispositif et de l'adapter à son utilisateur.

La prise de vues peut aussi être stéréoscopique, avec l'utilisation de deux ou N caméras pour correspondre à la disparité rétinienne de la vision binoculaire, et être restituée avec le dispositif adéquat (par sélection anaglyphique, par polarisation linéaire et circulaire, sur écran auto stéréoscopique, à l'aide d'une carte de profondeurs de l'image ('zmap'), par un système de vision « 2D augmentée » ou par tout autre système de restitution en relief ou volume de type holographique.

### Réalisation d'une commande oculaire pour diriger un mouvement de caméra 2D dans le plan de l'objectif

### Etape 1 - Sélection de la fonction « prise de vues »

La fonction « prise de vues » est activée soit par l'appui sur une touche clavier, soit par une pause oculaire sur la zone du bouton « prise de vues » soit encore par un ordre vocal. Le bouton « prise de vues » est mis en inverse vidéo, indicateur du mode actif. Cela signifie que le dispositif d'initialisation est enclenché: dorénavant, les mouvements lents et leurs directions asserviront la caméra.

### Etape 2 - Analyse en ligne des données : détection du mouvement lent par la technique d'apprentissage non supervisé ou semi-supervisé

Les données de base sont les positions XY de l'oeil gauche (G) et de l'oeil droit (D) sur l'écran, fournies en sortie par l'oculomètre, échantillonnées toutes les 20 millisecondes (T) comme dans le tableau 1 ci-dessous.

**Tableau 1. Données oculaires de base.**

| Référence | T | X | Y | X | Y |
|---|---|---|---|---|---|
| | | (G) | (G) | (D) | (D) |
| 1 | 19 | 506 | 376 | 520 | 376 |
| 2 | 39 | 501 | 381 | 521 | 372 |
| 3 | 59 | 502 | 378 | 515 | 372 |
| 4 | 79 | 502 | 379 | 516 | 370 |
| 5 | 99 | 505 | 385 | 516 | 391 |
| 6 | 118 | 507 | 377 | 512 | 380 |
| 7 | 138 | 502 | 384 | 518 | 372 |

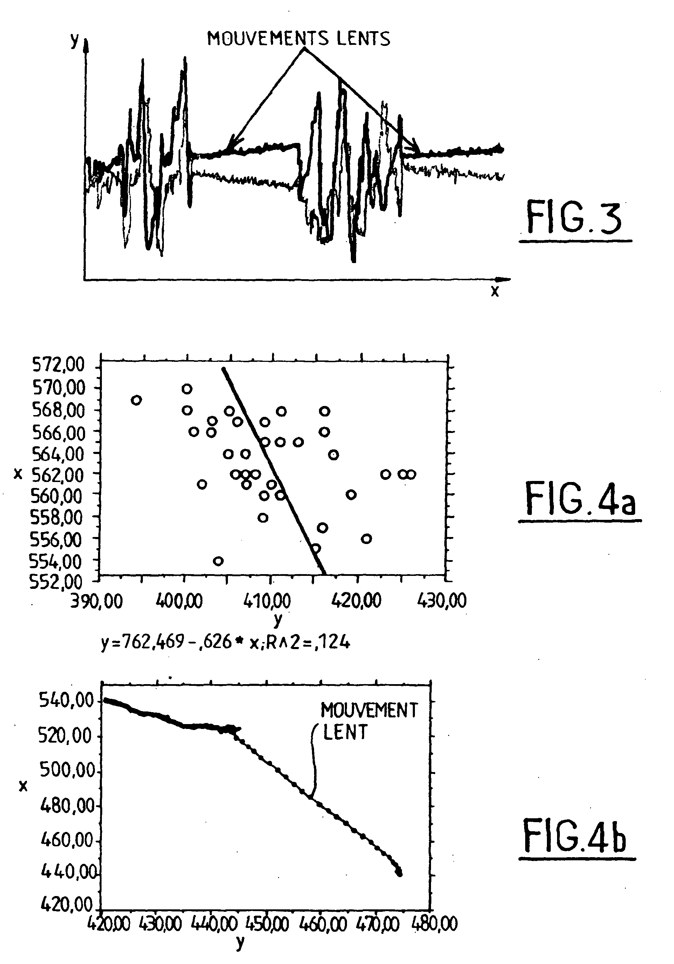

Le problème à résoudre consiste à repérer rapidement l'apparition de mouvements lents tels ceux de la figure 3.

Les données oculaires de base sont susceptibles de contenir 3 types de mouvements oculaires : les saccades, les pauses et les mouvements lents. Le principe d'analyse consiste à différencier ces trois types de données oculaires séquentielles.

A la différence des mouvements par saccades, les mouvements lents se composent de données séquentielles de valeurs proches les unes des autres (micro saccades). Ces données peuvent êtres organisées selon différents modèles comme par exemple un segment ayant une pente, cette pente étant, ici, déterminée par régression linéaire en raison de la relative dispersion des points qu'induit la succession des micro-saccades (voir la figure 4a dont l'échelle est agrandie). Les pauses oculaires, dont les données ont également des valeurs proches, s'en différencient par le fait que leurs données n'ont pas cette organisation selon un segment orienté.

Les données sont analysées dans des fenêtres temporelles successives de 500 millisecondes, soit 25 données chacune. Un algorithme d'apprentissage non supervisé définit si les deux ensembles de données, traitées en bloc, appartiennent à une même classe ou non. L'apprentissage non supervisé consiste à identifier des classes d'éléments possédant des caractéristiques aussi semblables que possible qui les différencient des éléments d'autres classes.

Les éléments sont ici des séquences de données appartenant à l'une des deux classes "mouvement lent" et "mouvement non lent". Les caractéristiques des données étant sujettes à une certaine variabilité et à des imprécisions, on peut utiliser:
- une méthode de classification vectorielle classique,
- une classification floue, dont le but est de traiter des degrés d'appartenance à des classes imprécisément définies, associée à l'utilisation de méthodes statistiques telles que l'analyse de la variance (on renforce les résultats par l'utilisation d'un petit nombre de données étiquetées à priori par "mouvement lent" ou "mouvement non lent" afin de pouvoir utiliser une méthode d'apprentissage semi-supervisé),
- ou par toute autre méthode de classification.

La présence de deux classes successivement est l'indicateur d'une saccade.

Ainsi, dans le cas de la régression linéaire, si deux ensembles de données correspondent à une classe, la corrélation XY permet de déterminer la pente de la droite de régression et de différencier entre pause oculaire et mouvement lent (figure 4b). Dans le cas du mouvement lent, le traitement des pentes successives permet alors de déterminer pour deux groupes de points successifs le mouvement de la caméra, car on connaît la direction du mouvement lent.

### Etape 3 - Corrections apportées au mouvement de la caméra par la technique d'apprentissage supervisé et par la commande.

Les ajustements de la vitesse du mouvement de la caméra sont réalisés par technique d'apprentissage supervisé comme, par exemple, un apprentissage inductif par arbre de décision. Elle consiste en l'utilisation d'exemples étiquetés, chaque exemple étant représenté par diverses caractéristiques et par une étiquette indiquant une action le concernant. Et elle s'effectue en déterminant automatiquement la pertinence des caractéristiques pour identifier l'action qui correspondra à un nouvel exemple dont on ne connaît que les caractéristiques: Cet apprentissage permet d'apprendre des règles liant le mouvement oculaire et le mouvement de la caméra. Les données étant ici encore imprécises ( plusieurs mouvements voisins de l'oeil pouvant, dans certains cas, conduire à un même mouvement de caméra) on utilisera, de préférence, une méthode d'apprentissage floue, comme, par exemple, un apprentissage par arbre de décision flou. Chaque commande étant paramétrable, l'utilisateur détermine la relation entre la vitesse d'exécution de la commande et le déplacement lent de son regard (point 10 de la description du procédé). La commande transmise à la caméra est corrigée à partir de ces paramètres.

Une méthode de commande floue comme la méthode de Takagi-Sugeno dont le principe est d'exploiter des règles floues (telles que celles obtenues par un arbre de décision flou) pour piloter un système par des actions précises (comme celles indiquées dans la table de correspondance au point 7) permet une telle correction de façon automatique.

D'autres méthodes utilisables d'apprentissage et de commande en logique floue sont décrites par exemple dans l'ouvrage de B. Bouchon-Meunier et C. Marsala 'Traitement de données complexes et commande en logique floue' Ed. Hermès-2003.

Le mouvement lent peut être utilisé pour effectuer une commande proportionnelle : la vitesse d'un zoom peut être, par exemple, fonction de la vitesse de déplacement de l'oeil. Cette commande peut être initiée:
- par le mouvement lent lui-même,
- par un ordre (vocal, manuel comme action sur un clavier, 'clic' d'une souris, action d'une manette de jeu, etc. ou bien encore visuel par fixation, par exemple, sur une icône).

Le mouvement lent peut être également utilisé en mode tout ou rien pour effectuer une commande déterminée à l'avance.

Le mouvement lent peut être utilisé pour sélectionner un type de commande. On peut, par convention, décider qu'un mouvement horizontal commandera un 'travelling horizontal', un panoramique ou bien encore un mouvement combiné de panoramique et de 'travelling' (le sens et la vitesse du mouvement décidant du sens et de la vitesse du 'travelling' et/ou du panoramique).

Le départ et/ou la fin de la commande peuvent être donnés respectivement par le début ou la fin du mouvement lent ou bien par un ordre vocal, visuel ou manuel.

### EXEMPLES D'APPLICATIONS :

### 1- Mouvements lents de commandes oculaires par mouvement lent pour caméras réelles ou virtuelles 2D ou 3D (relief).

### Caméras 2D réelles:

Le suivi d'un sujet à filmer peut s'appliquer à tous les axes de prise de vues et à toutes les fonctionnalités de la caméra.

Les axes sont:
- pour une grue de cinéma : travelling, montée ('boom') et rotation ('swing') du bras,
- pour une tête: rotation horizontale (panoramique 'pan'), verticale ('tilt') et autour
- de l'axe optique (`roll'),
   - pour une caméra équipée d'un zoom : zoom, mise au point et/ou diaphragme,
   - pour une caméra équipée d'accessoires : variation compensée de la vitesse de l'obturateur et/ou du diaphragme (`Variospeed')
   - et en général toute fonction de prise de vues intégrée au menu caméra.

Caméras 3D (relief) réelles: la vergence et l'entraxe. Asservissement de tout effet motorisé ou algorithmique sur les caméras: vitesse de fondu, etc.

Toutes ces fonctions peuvent être asservies par le regard et sont cumulables notamment de façon hiérarchisée ou non, en définissant le type de commande, par exemple, à l'aide d'un ordre manuel, visuel ou vocal.

**EXEMPLE :** suivi d'un match de football avec mouvement combiné de panoramique et de 'travelling' à partir d'un mouvement lent.

Décision préalable : le 'travelling' suit l'action à une vitesse pouvant aller jusqu'à 10 m/s (vitesse de la course humaine) avec limitation angulaire afin de contraindre le travelling à suivre l'action avec une amplitude de panoramique inférieure à, par exemple, 20°. Quand l'opérateur suit le ballon, le mouvement lent initie le panoramique. Dès que l'angle de rotation approche 20° (par exemple 15°),le 'travelling' se met automatiquement en fonction pour revenir à une position de perpendicularité par rapport à l'axe longitudinal du terrain.

### Caméras virtuelles 2D et 3D:

Toutes les fonctionnalités des caméras réelles sont applicables aux caméras virtuelles.

Sur le même mode (combinaisons de panoramiques et de 'travellings' à partir d'un mouvement lent), un spectateur visite un musée ou joue à un jeu vidéo en se déplaçant de salle en salle. Tous les axes peuvent être asservis et hiérarchisés, la seule limite à l'évolution du spectateur est constituée par le volume de l'environnement calculé (en images de synthèse).

### 2- Utilisation du mouvement lent pour choisir une valeur parmi plusieurs ou un continuum de valeurs.

**EXEMPLE** : Outil d'étalonnage dédié à la post-production. L'opérateur choisit du regard un point de l'image sur laquelle il désire travailler. Ce choix détermine une zone de l'image ayant des propriétés équivalentes au point choisi. Cette zone est alors sélectionnée. Ensuite, il promène son regard sur un diagramme de chromaticité 'CIE' (Commission Internationale d'Eclairage). Au cours de cette promenade du regard, les couleurs balayées s'affichent dans la zone sélectionnée. Une commande oculaire attribue la sélection à la zone.

L'opérateur peut ainsi sélectionner en continu, sans perdre l'image des yeux, la valeur (couleur ou densité) qui lui convient.

### 3- Mouvement lent de sélection

### EXEMPLE : sélection d'outils

### Outil réel :

Un opérateur à distance avec faculté de contrôle visuel (salles blanches) est amené à choisir différents outils de manipulation/travail pour opérer des actions sur des objets de natures (volumétriques) différentes. Du regard, il choisit dans une gamme l'outil adapté à la manipulation de l'objet, il en équipe son bras (commandé) et effectue son action.

La détection de l'arrêt du mouvement lent du regard sélectionne par conséquent le type d'outil adéquat.

### Outil virtuel :

Un joueur peut, sur le même principe, accéder à des instruments de différentes sortes sélectionnés par le regard.

### 4- Combinaison d'un mouvement lent et d'ordres annexes.

De la même façon que tout mouvement d'une caméra réelle ou virtuelle 2D ou 3D peut se combiner en fonction de la décision et de la hiérarchie choisie par l'opérateur, celui-ci peut décider à tout moment l'ordre de départ du mouvement de la caméra ou l'ordre d'arrêt, ou au milieu d'un 'travelling', ajouter un zoom ou un changement de vergence dans la profondeur du cadre.

Dans ce cas, l'ordre peut être manuel, lié à la gestion d'une icône ou vocal, ce qui permet de ne pas quitter l'action des yeux.

### 5- Prise en compte de la vitesse.

**EXEMPLE :** rotation autour de l'axe optique ('roll') dans une image de synthèse
- Le mouvement lent de basculement (rotation) de la tête de l'observateur indique à la fois, pour la rotation effectuée en correspondance à l'écran, le sens, la vitesse et l'accélération de la rotation produite. Cette rotation s'effectuant selon l'axe passant par le point fixé par l'opérateur sur l'écran et le point médian entre ses deux yeux.
- Le mouvement se poursuit sans intervention de l'opérateur jusqu'au moment où il décide de l'arrêter.
- S'agissant d'une caméra, il peut, avant l'action, choisir par le regard un certain nombre d'images de ralenti de début et/ou de fin.

### 6- Commandes liées aux réglages des paramètres du relief :

L'entraxe caméra peut augmenter ou diminuer en fonction de l'observation d'une échelle ou d'un barre-graphe qui fait suite à un ordre vocal ou au repérage d'une icône appropriée.

La vergence peut être gérée par le repérage visuel du plan de convergence. Ceci implique qu'il existe un repère visuel.

### 7- Suivi d'une scène réelle.

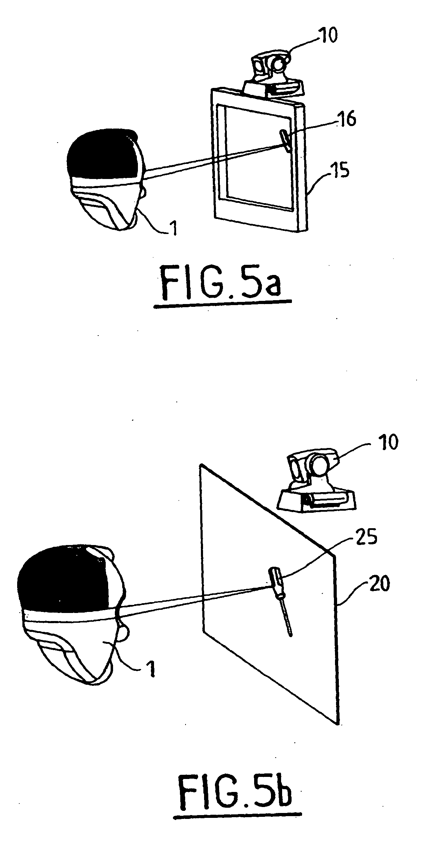

Le déplacement du regard est détecté par un oculomètre.
a. L'observateur regarde un moniteur ou une matrice active (Figure 5a).
   Le regard peut se déplacer sur un moniteur 15, pour diriger l'orientation de la caméra à commander pour cadrer un objet 16. Il peut ainsi diriger une télé-opération à distance quelle que soit cette distance.
b. L'observateur regarde une scène réelle à travers un verre à matrice active (Figure 5a).
   C'est l'exemple d'un match de football ou d'une pièce de théâtre.
   L'observateur poursuit l'action des yeux à travers le verre transparent 15 de façon à commander la caméra dont les paramètres (valeur de cadre, déplacement,...) s'affichent sur le verre. On peut faire apparaître, pour rendre compte de sa géométrie, l'image simplifiée de la scène réelle ou, pour rendre compte de sa profondeur, sa 'Zmap' ou encore une image 2D augmentée d'indices de profondeurs.
   Dans ce cas, la détection des mouvements du regard renvoie au sujet filmé.
   Un système d'affichage permet de visualiser le premier plan, le plan de convergence et le plan d'infini.
c. L'observateur regarde une scène réelle sans intermédiaire (Figure 5b).

Ici, il n'y a plus d'écran et on analyse directement le mouvement du regard porté sur une scène réelle 25 pour définir le cadre et l'axe caméra 20. Ce cadre et cet axe peuvent être visualisés sous forme d'image virtuelle.

Un assistant peut, par addition, régler la valeur de point, etc. en fonction de la scène suivie par l'opérateur. Ces réglages peuvent également être commandés par le regard.

Le dispositif peut donc fonctionner en cascade entre divers opérateurs, notamment pour l'opérateur qui commande sans écran tout ou partie des fonctions de la caméra.

Si ce sont l'orientation et l'angle de vergence qui sont détectés, toute commande en 2D et 3D est possible sans écran.

### 8- Exemple d'application concernant la gestion d'un outil en volume :

Commande d'un robot se déplaçant par commande du regard avec fonctions d'asservissement d'un outil à positionner et à commander.

Pour ce type de fonction, la stéréoscopie peut présenter un réel avantage pour manipuler ou usiner un objet de surface complexe.

Une détection des volumes du sujet traité et la modélisation de la machine sont nécessaires.

Un détecteur de positionnement interdit toute pénétration accidentelle et, s'il s'agit d'usinages, la distance et l'angle corrects entre l'outil et la surface à traiter sont connus.

## Revendications

1. Procédé de commande à partir d'un signal oculaire de commande, **caractérisé en ce qu'**il comporte les étapes suivantes :
- détecter au moins un mouvement lent correspondant à un déplacement oculaire pour au moins un oeil d'un opérateur,
- générer un dit signal oculaire de commande à partir d'au moins un mouvement lent détecté,
- produire une commande à partir du signal oculaire de commande, **caractérisé en ce qu'**il comporte une procédure préalable d'apprentissage affectée à un opérateur en analysant les données de position oculaire dans des fenêtres temporelles successives de même durée T et en classant les ensembles de données de chaque fenêtre dans au moins deux classes :
- une classe de mouvements lents pour laquelle un ensemble de données comporte des données séquentielles de valeurs proches les unes des autres organisées selon une forme géométrique continue, par exemple un segment ayant une pente,
- une classe de mouvements non lents sinon.

2. Procédé selon la revendication 1, **caractérisé en ce qu'**un dit mouvement lent correspond à un déplacement oculaire régulier dont la vitesse angulaire est comprise entre 1°/s et 40°/s, notamment entre 1°/s et 30°/s, et plus particulièrement entre 5°/s et 20°/s.

3. Procédé selon une des revendications 1 ou 2, **caractérisé en ce que** la détection du mouvement lent comprend la détermination de son trajet, notamment de sa direction par un procédé de calcul, notamment par régression linéaire.

4. Procédé selon une des revendications précédentes, **caractérisé en ce qu'**une détection d'une saccade et/ou d'une fixation oculaire interrompt la génération du signal oculaire de commande.

5. Procédé selon une des revendications précédentes, **caractérisé en ce que** le signal oculaire de commande est une fonction du déplacement oculaire.

6. Procédé selon la revendication 5, **caractérisé en ce que** le signal oculaire de commande représente le point de l'espace où l'oeil se situe et/ou la vitesse du mouvement lent de l'oeil et/ou sa direction.

7. Procédé selon une des revendications 5 ou 6, **caractérisé en ce qu'**au moins un paramètre de la commande, par exemple une vitesse, est fonction d'au moins un paramètre du déplacement oculaire, par exemple sa vitesse et/ou sa direction.

8. Procédé selon la revendication 5 ou 6, **caractérisé en ce que** la commande consiste en un choix selon un continuum ou un choix entre un nombre discret d'options, en fonction d'au moins un paramètre du déplacement oculaire.

9. Procédé selon une des revendications 1 à 4, **caractérisé en ce que** le signal oculaire de commande est un signal par tout ou rien.

10. Procédé selon une des revendications précédentes, **caractérisé en ce que** le type de commande auquel le signal oculaire de commande est affecté est déterminé par au moins un paramètre du déplacement oculaire, par exemple sa direction.

11. Procédé selon une des revendications précédentes, **caractérisé en ce que** le type de commande auquel le signal oculaire de commande est affecté est déterminé par un ordre vocal, visuel ou manuel.

12. Procédé selon une des revendications précédentes, **caractérisé en ce que** le début et/ou la fin de la commande est généré par le début et/ou la fin du signal oculaire de commande.

13. Procédé selon une des revendications précédentes, **caractérisé en ce que** le début et/ou la fin de la commande est généré par un ordre vocal, visuel ou manuel.

14. Procédé selon une des revendications précédentes **caractérisé en ce qu'**il est appliqué à la commande oculaire d'au moins une caméra.

15. Procédé selon la revendication 14, **caractérisé en ce qu'**il met en oeuvre au moins un type de commande choisi parmi: prise de vue et/ou zoom et/ou diaphragme et/ou mise au point et/ou mouvement panoramique et/ou rotation de la caméra autour de son axe et/ou déplacement (« travelling »).

16. Procédé selon une des revendications 14 ou 15, **caractérisé en ce qu'**il est appliqué à la commande oculaire d'un système de prise de vue stéréoscopique comportant au moins deux caméras.

17. Procédé selon la revendication 16, **caractérisé en ce qu'**il met en oeuvre au moins un type de commande choisi parmi : entraxe et/ou vergence.

18. Procédé selon la revendication 1, **caractérisé en ce que** la classe de mouvements non lents comporte deux sous-classes :
- une sous-classe de fixation oculaire comportant des données de valeurs proches mais qui ne sont pas organisées selon une forme géométrique, par exemple un segment ayant une pente,
- et une sous-classe de saccades oculaires sinon.

## Claims

1. A control method based on a controlling eye signal, the method being **characterized in that** it comprises the following steps:
detecting at least one slow movement corresponding to an eye movement of at least one eye of an operator;
generating a said eye control signal from at least one detected slow movement; and
producing a control from the eye control signal, **characterized in that** it includes a prior training procedure performed with an operator in which eye position data in successive same-duration (T) time windows is analyzed by classifying data sets in each window in each two classes:
a class of slow movements for which a data set comprises sequential data points of values that are close to one another and organized in a geometrical shape that is continuous, e.g. a segment having a slope; and
otherwise a class of non-slow movements.

2. A method according to claim 1, **characterized in that** a said slow movement corresponds to a regular eye movement of angular speed lying in the range 1°/s to 40°/s, in particular in the range 1°/s to 30°/s, and more particularly in the range 5°/s to 20°/s.

3. A method according to claim 1 or claim 2, **characterized in that** the detection of the slow movement comprises determining its path, in particular its direction, by a calculation method, in particular by linear regression.

4. A method according to any preceding claim, **characterized in that** detecting a saccade and/or a fixed gaze interrupts generation of the eye control signal.

5. A method according to any preceding claim, **characterized in that** the eye control signal is a function of eye movement.

6. A method according to claim 5, **characterized in that** the eye control signal represents the point in three dimensions where the eye is situated and/or the speed of the slow movement of the eye and/or its direction.

7. A method according to claim 5 or claim 6, **characterized in that** at least one control parameter, e.g. a speed, is a function of at least one parameter of the eye movement, e.g. its speed and/or directions.

8. A method according to claim 5 or claim 6, **characterized in that** the control consists in making a choice on a continuum or a choice amongst a discrete number of options, as a function of at least one parameter of the movement of the eye.

9. A method according to any one of claims 1 to 4, **characterized in that** the eye control signal is an on/off signal.

10. A method according to any preceding claim, **characterized in that** the type of control to which the eye control signal is allocated is determined by at least one parameter of eye movement, e.g. its direction.

11. A method according to any preceding claim, **characterized in that** the type of control to which the eye control signal is allocated is determined by an order given by voice, by eye, or by hand.

12. A method according to any preceding claim, **characterized in that** the beginning and/or the end of the control is generated by the beginning and/or the end of the eye control signal.

13. A method according to any preceding claim, **characterized in that** the beginning and/or the end of the control is generated by an order given by voice, by eye, or by hand.

14. A method according to any preceding claim, **characterized in that** it is applied to controlling at least one camera by eye.

15. A method according to claim 14, **characterized in that** it implements at least one type of control selected from: picture-taking and/or adjusting zoom and/or adjusting aperture and/or adjusting focus and/or panning and/or rolling the camera about its own axis and/or traveling.

16. A method according to claim 14 or claim 15, **characterized in that** it is applied to controlling by eye a stereoscopic picture-taking system that includes at least two cameras.

17. A method according to claim 16, **characterized in that** it implements at least one type of control selected from: spacing between axes and/or vergence.

18. A method according to claim 1, **characterized in that** the class of non-slow movements comprises two subclasses:
an eye gaze sub-class comprising data points of values that are close but that are not organized in a symmetrical shape, e.g. a segment having a slope; and
otherwise a sub-class of eye saccades.

## Patentansprüche

1. Steuerungsverfahren, ausgehend von einem okularen Steuersignal, **dadurch gekennzeichnet, dass** es die nachfolgenden Schritte umfasst:
- Erfassen zumindest einer langsamen Bewegung, die einer Augenbewegung von zumindest einem Auge einer Bedienperson entspricht,
- Erzeugen eines okularen Steuersignals, ausgehend von zumindest einer erfassten langsamen Bewegung,
- Ausführen einer Steuerung, ausgehend vom okularen Steuersignal,
**dadurch gekennzeichnet, dass** es einen vorhergehenden Lernprozess umfasst, der einer Bedienperson zugeordnet ist und bei dem die Daten der Augenstellung in aufeinanderfolgenden Zeitfenstern gleicher Zeitdauer T analysiert und die Datensätze eines jeden Fensters in zumindest zwei Klassen eingeteilt werden:
- eine Klasse von langsamen Bewegungen, bei der ein Datensatz sequentielle Daten mit nahe beieinanderliegenden Werten aufweist, die gemäß einer kontinuierlichen geometrischen Form angeordnet sind, beispielsweise gemäß einem Segment mit einer Schräge,
- eine Klasse von sonstigen nicht langsamen Bewegungen.

2. Verfahren nach Anspruch 1, **dadurch gekennzeichnet, dass** eine langsame Bewegung einer gleichmäßigen Augenbewegung entspricht, deren Winkelgeschwindigkeit zwischen 1°/s und 40°/s, insbesondere zwischen 1 °/s und 30°/s und im besonderen zwischen 5°/s und 20°/s liegt.

3. Verfahren nach einem der Ansprüche 1 oder 2, **dadurch gekennzeichnet, dass** die Erfassung der langsamen Bewegung die Bestimmung ihrer Wegstrecke, insbesondere ihrer Richtung, durch ein Rechenverfahren, insbesondere durch lineare Regression, umfasst.

4. Verfahren nach einem der vorangehenden Ansprüche, **dadurch gekennzeichnet, dass** durch Erfassen einer Sakkade und/oder einer Fixierung der Augen das Erzeugen des okularen Steuersignals unterbrochen wird.

5. Verfahren nach einem der vorangehenden Ansprüche, **dadurch gekennzeichnet, dass** das okulare Steuersignal eine Funktion der Augenbewegung ist.

6. Verfahren nach Anspruch 5, **dadurch gekennzeichnet, dass** das okulare Steuersignal den Punkt im Raum, wo sich das Auge befindet, und/oder die Geschwindigkeit der langsamen Bewegung des Auges und/oder deren Richtung darstellt.

7. Verfahren nach einem der Ansprüche 5 oder 6, **dadurch gekennzeichnet, dass** zumindest ein Parameter bei der Steuerung, beispielsweise eine Geschwindigkeit, eine Funktion von zumindest einem Parameter der Augenbewegung ist, beispielsweise von ihrer Geschwindigkeit und/oder ihrer Richtung.

8. Verfahren nach Anspruch 5 oder 6, **dadurch gekennzeichnet, dass** die Steuerung aus einer Auswahl gemäß einem Kontinuum oder aus einer Auswahl zwischen einer diskreten Anzahl von Optionen in Abhängigkeit von zumindest einem Parameter der Augenbewegung besteht.

9. Verfahren nach einem der Ansprüche 1 bis 4, **dadurch gekennzeichnet, dass** das okulare Steuersignal ein Ein-Aus-Signal ist.

10. Verfahren nach einem der vorangehenden Ansprüche, **dadurch gekennzeichnet, dass** die Art von Steuerung, dem das okulare Steuersignal zugeordnet ist, von zumindest einem Parameter der Augenbewegung, beispielsweise von ihrer Richtung, bestimmt wird.

11. Verfahren nach einem der vorangehenden Ansprüche, **dadurch gekennzeichnet, dass** die Art von Steuerung, dem das okulare Steuersignal zugeordnet ist, durch einen vokalen, visuellen oder manuellen Befehl bestimmt wird.

12. Verfahren nach einem der vorangehenden Ansprüche, **dadurch gekennzeichnet, dass** der Beginn und/oder das Ende der Steuerung durch den Beginn und/oder das Ende des okularen Steuersignals erzeugt wird.

13. Verfahren nach einem der vorangehenden Ansprüche, **dadurch gekennzeichnet, dass** der Beginn und/oder das Ende der Steuerung durch einen vokalen, visuellen oder manuellen Befehl erzeugt wird.

14. Verfahren nach einem der vorangehenden Ansprüche, **dadurch gekennzeichnet, dass** es bei der okularen Steuerung zumindest einer Kamera Anwendung findet.

15. Verfahren nach Anspruch 14, **dadurch gekennzeichnet, dass** zumindest eine Art von Steuerung durchgeführt wird, ausgewählt aus: Aufnahme und/oder Zoom und/oder Blende und/oder Scharfstellung und/oder Panoramaschwenk und/oder Drehung der Kamera um ihre Achse und/oder Verlagerung ("travelling").

16. Verfahren nach einem der Ansprüche 14 oder 15, **dadurch gekennzeichnet, dass** es bei der okularen Steuerung eines stereoskopischen Aufnahmesystems mit zumindest zwei Kameras Anwendung findet.

17. Verfahren nach Anspruch 16, **dadurch gekennzeichnet, dass** zumindest eine Art von Steuerung durchgeführt wird, ausgewählt aus: Achsabstand und/oder Lichtbrechungsvermögen.

18. Verfahren nach Anspruch 1, **dadurch gekennzeichnet, dass** die Klasse von nicht langsamen Bewegungen zwei Unterklassen enthält:
- eine Unterklasse okularer Fixierung nach einer geometrischen Form, beispielweise einem Segment mit einer Schräge,
- und eine Unterklasse sonstiger okularer Sakkaden.
